# EUROPEAN PATENT APPLICATION

(11) **EP 1 992 358 A1**
(43) Date of publication of application: **19.11.2008**
(21) Application number: 07711005.4
(22) Date of filing: 16.02.2007
(51) Int. Cl.: A61K 39/21, A61K 39/285, C12N 15/49, C12N 15/863, A61P 31/18

(54) **AIDS VACCINE BASED ON REPLICATIVE VACCINIA VIRUS VECTOR**

(30) Priority: 16.02.2006 CN 200610007567
(71) Applicant: Chinese Center for Disease Control and Prevention Center for Aids/STD Control and Prevention, Beijing 100050 (CN)
(72) Inventor: SHAO, Yiming, Beijing 100050 (CN); LIU, Ying, Beijing 100050 (CN); LIU, Jianyuan, Beijing 100024 (CN)
(74) Representative: Hatzmann, Martin
(86) International application number: PCT/CN2007/000589
(87) International publication number: WO 2007/093134

(57) **Abstract**

Replicative live-vector vaccine against AIDS expressing antigen of human immunodeficiency virus (HIV) and its application are provided. Said vaccine is constructed on the basis of replicative vaccinia virus, such as vaccinia virus TianTan strain. The replicative live-vector vaccine of present invention is capable of inducing high level humoral and cell immunoresponse against HIV. The vector for constructing said vaccine, and immunization procedure using said vaccine are also provided.

Sequence listing

<110>  National center for AIDS/STD Control and Prevention, Chinese Center for
Disease Control and Prevention

<120>  AIDS Vaccine Based on Replicative Vaccinia Virus Vector

<130>  I200501572CB

<160>  7

<170>  PatentIn version 3.2

<210>  1
<211>  2145
<212>  DNA
<213>  Human immunodeficiency virus

<400>  1
atgagagtga cggggatcag gaagaattat cggcatttat ggagatgggg caccatgctc      60

cttgggatgt tgatgatcag tagtgctgta ggaaacttgt gggtcacagt ctattatggg     120

gtacctgtat ggaaagaggc aaccaccact ttattttgtg catcagatgc taaagcatat     180

gatacagagg tacataatgt ttgggctaca catgcctgtg tacccgcaga ccccaaccca     240

caagaaatgg ttttggaaaa tgtaacagaa aattttaaca tgtggaaaag tgaaatggta     300

aatcagatgc aggaagatgt aatcagttta tgggatcaaa gcctaaaacc atgtgcaaag     360

ttgaccccac tctgtgtcac tttagaatgt agaaatgtta gcagtaatag taatgatacc     420

taccatgaga cctaccatga gagcatgagg gaaatgaaaa attgcccttt caatgcaacc     480

acagtagtaa gagataggaa gcagacagtg tatgcacttt tctatagact tgatatagta     540

ccacttacta agaagaacta tagtgagaat tctagtgagt attatagatt aataaattgt     600

aatacctcag ccataacaca agcctgtcca aaggtcactt ttgatccaat tcctatacac     660

tattgcactc cagctggtta tgcaattcta aagtgtaatg ataagatatt caatgggaca     720

ggaccatgcc ataatgttag cacagtacaa tgtacacatg ggattaagcc agtggtatca     780

actcaactac tgttaaatgg tagcctagca gaaggagaaa taataattag atctgaaaat     840

ctgacaaaca atgtcaaaac aataatagta catcttaatc aatctgtaga aattgtatgt     900

acaagacccg gcaataatac aagaaaaagt ataaggatag gaccaggaca aacattctat     960

gcaacaggag acataatagg agacataaga caagcacatt gtaacattag tgaagataaa    1020

tggaatgaaa ctttacaaag ggtaagtaaa aaattagcag aacacttcca gaataaaaca    1080

ataaaatttg catcatcctc aggaggggac ctagaagtta caacacatag ctttaattgt    1140

agaggagaat tcttctattg taatacatca ggcctgttta atggtacata cacgcctaat    1200

ggtacaaaag gtaattcaag ctcaatcatc acaatcccat gcagaataaa gcaaattata    1260

aatatgtggc aggaggtagg acgagcaatg tatgcccctc ccataaaagg aaacataaca    1320

tgtaaatcaa atatcacagg actactattg gtacgtgatg gaggaacaga gccaaatgat    1380

acagagacat tcagacctgg aggaggagat atgaggaaca attggagaag tgaattatat    1440

aaatataaag tggtagaaat taagccattg ggagtagcac ccactacagc aaaaaggaga    1500

gtggtggaga gagaaaaaag agcagtggga ataggagctg tgttccttgg gttcttggga 1560

gtagcaggaa gcactatggg cgcggcgtca ataacgctga cggtacaggc cagacaattg 1620

ctgtctggta tagtgcaaca gcaaagcaat ttgctgaggg ctatagaggc gcaacagcat 1680

ctgttgcaac tcacggtctg gggcattaag cagctccaga caagagtcct ggctatagaa 1740

agatacctaa aggatcaaca gctcctaggg atttggggct gctctggaaa actcatctgc 1800

actactgctg taccttggaa ctccagttgg agtaacaaat ctcaaaaaga gatttgggat 1860

aacatgacct ggatgcaatg ggataaagaa attagtaatt acacaaacac aatatacagg 1920

ttgcttgaag aatcgcaaaa ccagcaggaa aggaatgaaa aagatctatt agcattggac 1980

agttggaaaa atctatggag ttggtttgac ataacaaatt ggctgtggta tataaaaata 2040

ttcataataa tagtaggagg cttgataggt ttaagaataa tttttgctgt gctctctata 2100

gtaaatagag ttaggcaggg atactgacct tagtcggatc cgaca 2145

<210> 2
<211> 2887
<212> DNA
<213> Human immunodeficiency virus

<400> 2
atgggtgcga gagcgtcaat attaagaggg ggaaaattag ataaatggga aaaaattagg 60

ttaaggccag ggggaaagaa acactatatg ctaaaacacc tagtatgggc aagctgggag 120

ctggaaagat ttgcacttaa ccctggcctt ttagagacat cagaaggctg taaacaaata 180

atgaaacagc tacaaccagc tcttcagaca ggaacagagg aacttagatc attattcaac 240

acagtagcaa ctctctattg tgtacataca gagatagatg tacgagacac caaagaagcc 300

ttagacaaga tagaggaaga acaaaacaaa attcagcaaa aaacacagca ggcaaaggag 360

gctgacggga aggtcagtca aaattatcct atagtacaga atctccaagg gcaaatggta 420

catcagccca tatcacctag aactttaaat gcatgggtaa aagcggtaga agagaaggct 480

tttagcccag aagtaatacc catgttttca gcgttatcag aaggagccac cccacaagat 540

ttaaacacca tgctaaacac agtgggggga catcaagcag ccatgcaaat attaaaagat 600

accatcaatg aagaggctgc agaatgggat agattacatc cagtacatgc agggcctatt 660

gcaccaggcc aaatgagaga accaagggga agtgacatag caggaactac tagtaaccta 720

caggaacaaa tagcatggat gacgagtaac ccacctgttc cagtaggaga catctataaa 780

agatggataa ttctgggatt aaataaaata gtaagaatgt atagccctac cagcattctg 840

gacataaaac aagggccaaa ggaacccttt agagactatg tagaccggtt ctttaaaact 900

ttaagagcgg aacaagctac acaagatgta aaaaattgga tgacagacac cttgttggtc 960

caaaatgcga acccagattg taagaccatt ttaagagcat taggaccagg ggcttcaata 1020

gaagaaatga tgacagcatg tcagggagtg ggaggaccta gccataaagc aaaagtgttg 1080

gccgaggcaa tgagccaaac aaacagtgcc atactgatgc agagaagcaa ttttaaaggc 1140

tctaaaagaa ttgttaaatg tttcaactgt ggcaagggag ggcacatagc cagaaattgc 1200
agggccccta ggaaaaaggg ctgttggaaa tgtggaaaag aaggacacca aatgaaagat 1260
tgtactgaga gacaggccaa ttttttaggg aaaatctggc cctcccacaa gggaaggcca 1320
gggaattttc ttcagaacag accagagcca acagccccac cagaggagag cttcaggttt 1380
ggggaagaga caacaactcc atctcagaag caggagccaa tagacaagga actatatcct 1440
ttaacttccc tcaaatcact ctttggcaac gacccctcgt cacaataaag ataggggggc 1500
aattaaagga agctctatta gatacaggag cagatgatac agtattagaa gacctgaatt 1560
tgccagggaa atggaaacca aaaatgatag ggggaattgg aggttttatc aaagtaagac 1620
agtatgaaca gatacccata gaaatttgcg gacacaaagc tataggtaca gtattagtag 1680
gacctacacc tgtcaacata attggaagaa atctgttgac tcagcttggt tgcactttaa 1740
attttccaat cagtcccatt gaaactgtac cagtaaaatt aaagccagga atggatggcc 1800
caaaggttaa acaatggcca ttgacagaag agaaaataaa agcattaaca gcaatttgtg 1860
atgaaatgga gaaagaagga aaaattacaa aaattgggcc tgaaaatcca tataacactc 1920
caatatttgc cataaaaaag aaggacagta ctaagtggag aaagttagta gatttcaggg 1980
aactcaataa aagaactcaa gatttttggg aagttcaatt aggaatacca cacccagcag 2040
ggttaaaaaa gaaaaaatca gtgacagtac tggatgtggg ggatgcatat ttttcaattc 2100
ctttatatga agacttcagg aagtatactg cattcaccat acctagtaga aacaatgaaa 2160
caccagggat taggtatcag tacaatgtac ttccacaggg atggaaagga tcaccagcaa 2220
tattccaaag tagcatgaca aaaatcttag agccttttag aaaacaaaat ccagacatag 2280
ttatctatca atacatggat gatttgtatg taggatctga cttagagata gggcagcata 2340
gaacaaaaat agaggaactg agacaacatt tgttgaggtg gggatttacc acaccagaca 2400
agaaacatca gaaagaacct ccatttcttt ggatggggta tgaactccat cctgacaaat 2460
ggacagtaca gcctatacag ctgccagaaa aagatagctg gactgtcaat gatatacaaa 2520
agttagtggg aaaattaaac tgggcaagtc agatttatcc tggaattaaa gtaaggcaac 2580
tttgtaaact ccttaggggg gccaaagcac taacagacat agtaccacta actgaagaag 2640
cagaattaga attggcagaa aacagggaaa ttctaaaaga accagtacat ggagtatact 2700
atgacccatc aaaagacttg atagctgaaa tacagaaaca ggggcaggaa caatggacat 2760
atcaaattta ccaagaacca ttcaaaaatc taaaaacagg gaagtatgca aaaatgagga 2820
ccgcccacac taatgatgta aaacaattaa cagaggctgt gcagaaaata gccatggaag 2880
gctagta 2887

<210> 3
<211> 25
<212> DNA
<213> Artificial

<220>
<223> Gagpol-for

<400> 3
gcggaggcta gaaggagaga gatgg 25

<210> 4
<211> 30
<212> DNA
<213> Artificial

<220>
<223> Gagpol-rev

<400> 4
ctactagcct tccatggcta ttttctgcac 30

<210> 5
<211> 30
<212> DNA
<213> Artificial

<220>
<223> Gp140-for

<400> 5
agcggatcca ccatgagagt gacggggatc 30

<210> 6
<211> 28
<212> DNA
<213> Artificial

<220>
<223> Gp140-rev

<400> 6
gtcggatccg actaaggtca gtatcctg 28

<210> 7
<211> 429
<212> DNA
<213> Artificial

<220>
<223> LacZ'-PE6

<400> 7
gccccgggct cgagttatga tctacttcct taccgtgcaa taaattagaa tatattttct 60
acttttacga gaaattaatt attgtattta ttatttatgg gtgaaaaact tactataaaa 120
agcgggtggg tttggaatga tgtaaagctt aaaaattgaa attttatttt ttttttttgg 180
aatataaata agctcgaagt cgacgcccaa ctggtaatgg tagcgaccgg cgctcagctg 240
gaattccgcc gatactgacg ggctccagga gtcgtcgcca ccaatcccca tatggaaacc 300
gtcgatattc agccatgtgc cttcttccgc gtgcagcaga tggcgatggc tggtttccat 360
cagttgctgt tgactgtagc ggctgatgtt gaactggaag tcgccgcgcc actggtgtgg 420
gccatgttt 429

## Description

### Field of Invention

This invention relates to antiviral immunology. More specifically, the invention involves a vaccine against Human Immunodeficiency Virus (HIV) based on a replicative vaccinia virus vector and its preparation methods and application. The AIDS vaccine of the invention can induce high level of humoral and cellular immune response against HIV.

### Background of Invention

Acquired Immunodeficiency Syndrome (AIDS) is an infectious disease caused by the Human Immunodeficiency Virus (HIV). AIDS has spread with a tremendous speed globally ever since the first case was identified in 1981, posing a serious threat to human health and human life as one of the most severe viral diseases in existence. The spreading of AIDS has greatly impacted the worldwide social and economic development. HIV infection has shortened the average life span, decreased the size of the labor force, and caused food shortages in some developing countries, reversing their economic and social development by 20 years. The HIV epidemic in China can be divided into three phases: an introduction phase (1985-1988), spread phase (1989-1994), and rapid spread phase (1995 till present). In recent years, the HIV epidemic has become more and more serious. By the end of 2003, the estimated HIV infected population has reached 840,000 in China, with an overall infection rate of 0.07%. Cumulatively, the number of people that died of AIDS has reached 160,000. Currently the HIV infection rate in China ranks 14^{th} in the world and 2^{nd} in Asia. Moreover, the number of HIV infected people is increasing at an annual rate of 40%.

Great progress has been made in the development and application of anti-HIV agents, offering better treatment to some AIDS patients. However, the occurrence of resistant strains has rendered some antiviral treatment ineffective to certain patients. The complicated lifelong therapy has made it difficult for patients to take the drugs on time, leading to treatment failure. High cost is another important factor that limits the application of such therapy in a larger scale. History has shown that the most economic and efficient way to control infectious diseases is the use of vaccines, such as the small pox vaccine, polio vaccine, measles vaccine and hepatitis vaccine. Therefore, the development of a safe and effective AIDS vaccine is the ultimate goal for AIDS researchers.

Many researchers agree that an effective AIDS vaccine should be able to elicit HIV-specific CD8+ cytotoxic T lymphocyte (CTL) response and neutralizing antibodies. However, reaching this goal with a single vaccine has proven difficult. A combination of different kinds of vaccines is needed to improve immune responses. The available candidate vaccines include: traditional vaccine (inactivated vaccine and attenuated live vaccine), synthetic peptide and protein subunit vaccine, DNA vaccine, and live vector vaccine.

Compared to other vaccines, the advantages of live vector vaccine are: (1) it is able to infect target tissue or cells, improving the efficiency of foreign genes entering cells; (2) the vector has an adjuvant effect, which can induce the production of cytokines and chemokines; and (3) most of the live vector vaccines can induce a durable immune response.

Among the live vector vaccines, the vaccinia virus-based vaccine has been extensively studied. However, most candidate vaccines tested in clinical trials, e.g., MVA, NYVAC, and ALVAC, are non-replicative vector based. The results of clinical trials show that the immunogenicity of non-replicative vector is relatively weak, and the immune responses induced in human were weaker than that in monkeys. The reason may be correlated to its non-replication nature. After infecting the host cells, the non-replicative vector can finish only one cycle of antigen expression, proceeding, and presentation, and does not have the ability to produce infective viruses to initiate a subsequent round of infection. Thus, the stimulations of non-replicative vector to the host immune system are limited and temporary. In light of this, vaccine research focus has turned from non-replicative vectors to replicative vectors, which can replicate after infection and elicit stronger immune responses in the body.

### Summary of Invention

This invention provides a new vaccine and vaccination method against HIV infection, wherein a replicative vaccinia virus is used as a vector to construct a live vector AIDS vaccine which expresses the HIV antigen.

A live vector AIDS vaccine expressing an HIV antigen(s), which vaccine comprises a replicative vaccinia virus as vector, wherein at least one polynucleotide encoding the HIV antigen(s) is inserted into the thymidine kinase gene (TK) region of the vaccinia virus genome.

The replicative vaccinia virus used in this invention is, for example, the vaccinia virus Tiantan strain (VTT). Preferably, the recombinant replicative vaccinia virus is free of any selection marker genes.

In a preferred embodiment of the vaccine of the invention, said at least one polynucleotide encoding the HIV antigen(s) is from HIV strain 97CN54 which is the predominant prevalence HIV strain in China.

The HIV antigen that can be used to construct the vaccine of this invention can be, for example, gag-pol and/or gp140TM. Accordingly, in a specific embodiment of the invention, the present invention provides a live vector AIDS vaccine, which is constructed by inserting the gagpol and gp140TM genes of HIV strain 97CN54 into the TK region of vaccinia virus Tiantan strain.

Preferably, the polynucleotide encoding the HIV antigen may be subjected to modifications, for example, by synonymous mutation and/or partial deletion. The vaccine of the invention may further comprise a pharmaceutically acceptable adjuvant and/or carrier.

This invention also provides an anti-AIDS vaccination kit, which comprises a plurality of components and an instruction indicating the immunization regime, wherein one component is the vaccine of this invention.

This invention further provides a method for preventing or treating HIV infection in a subject comprising administering to the subject an effective amount of the vaccine of this invention.

### Brief Description of the Drawings

Fig. 1 shows the construction scheme of shuttle plasmid pVTT1.0.
Fig. 2 shows the results of restriction enzyme digestion of plasmid pVTT1.0 using KpnI, NdeI, and EcoRV. Lane M: MarkerDL15000; lane 1: pVTT1.0 digested with KpnI; lane 2: pVTT1.0 digested with NdeI; Lane3: pVTT1.0 digested with EcoRV.
Fig. 3 shows the results of restriction enzyme digestion of plasmid pVTT-gagpol using PstI, XbaI, and NcoI followed by electrophoresis. Lane 1: λDNA/HindIII+EcoRI marker; lane 2: pVTT-gagpol/PstI; lane 3: pVTT-gagpol/XbaI; lane 4: pVTT-gagpol/NcoI; lane 5: λDNA/HindIII marker; lane 6:DL15000 marker.
Fig. 4 shows the results of restriction enzyme digestion of plasmid pVTT-mgp140TM using NdeI and SalI followed by electrophoresis. Lane 1: marker DL2000; lane 2: pVTT-mgp140TM; lane 3: pVTT-mgp144TM/NdeI; lane 4: pVTT-mgp140TM/SalI
Fig. 5 shows the construction scheme of shuttle plasmid pVTT-gagpolenv. The plasmid pVTT-gagpol was digested with PmeI and PacI, filled in to form blunt ends and recovered from agarose gel to obtain a 3.0kb fragment of pE/L-gagpol. The plasmid pVTT-mgp140TM was digested with PmeI, and then ligated with pE/L-gagpolΔ.
Fig. 6 shows the results of restriction enzyme digestion of plasmid pVTT-gagpolenv using HindIII and pstI followed by electrophoresis. Lane 1: pVTT-gagpolenv/HindIII; lane 2: pVTT-gagpolenv/pstI; lane 3: Marker DL2000; lane 4, Marker DL15000.
Fig. 7 shows the screening strategy of the recombinant virus.
Fig. 8 shows the antibody staining assay for screening the recombinant virus. A: plaque formation by Tiantan strain (negative); B: plaque formation by Tiantan strain expressing HIV antigen.
Fig. 9 shows the identification of VTKgagpolenv by PCR amplification. Lane1: VTKgagpolenv/gagpol; Lane 2: Tiantan strain/gagpol; Lane 3: VTKgagpolenv/gp140TM; Lane 4: Tiantan strain/gp140TM; Lane 5: Marker DL2000.
Fig. 10 shows the identification of VTKgagpolenv by Western Blotting. Lane 1: BENCHMARK^{™} Prestained protein Ladder; Lanes 2 and 3: cells infected with TKgagpolenv.
Fig. 11 is the results of Dot Blotting assay showing the absence of the selection marker. Lane 1: VTKgagpolenv; Lane 2: vaccinia virus Tiantan strain (negative control); 3. pVI-gagpol (positive control).
Fig. 12 shows the HIV-specific immune response induced by TKgagpolenv immunization in rabbits.
Fig. 13 shows the Env-specific cellular immune response induced by DNA priming/TKgagpolenv boosting in Chinese rhesus macaques.
Fig. 14 shows the Gag-specific cellular immune response induced by DNA priming/TKgagpolenv boosting in Chinese rhesus macaques.
Fig. 15 shows the HIV-specific humoral immune response induced by DNA priming/TKgagpolenv boosting in Chinese rhesus macaques.
Fig. 16 shows the protection effects of the recombinant vaccinia virus Tiantan strain (rTV) vaccine in SHIV challenging test.

### Deposits

Plasmid pVTT1.0 was deposited in China General Microbiological Culture Collection Center (CGMCC) on Sep 19, 2005 under the deposit No. CGMCC No. 1458.

Plasmid pSC65 was deposited in China General Microbiological Culture Collection Center (CGMCC) on Feb 24, 2004 under the deposit No. CGMCC No. 1097.

E. coli strain PT-gp140TM/DH5α was deposited in China General Microbiological Culture Collection Center (CGMCC) on Aug 18, 2005 under the deposit No. CGMCC No. 1439.

E.coli strain PT-gagpol/DH5α was deposited in China General Microbiological Culture Collection Center (CGMCC) on Aug 18, 2005 under the deposit No. CGMCC No. 1438.

Monkey-Human chimeric virus SHIV-CN97001 was deposited in China General Microbiological Culture Collection Center (CGMCC) on Feb 14, 2007 under the deposit No. CGMCC No. 1945.

### Detailed Description

The anti-AIDS vaccine of the invention is developed based on replicative vaccinia virus vector, which is different from the non-replicative vaccinia virus vectors, such as MVA, NYVAC, and ALVAC. In the vaccine of the invention, at least one polynucleotide encoding an HIV antigen(s) was inserted into the TK region of the replicative vaccinia virus. The vaccine can induce protective immune responses against HIV upon administering to a subject.

The term "replicative" means the vaccinia virus vector is capable of replicating in the human body. In a preferred embodiment of this invention, the replicative vaccinia virus vector is vaccinia virus Tiantan strain (VTT). VTT was isolated in the 1920s by Chinese scientists, and was utilized widely in nationwide vaccinations against smallpox in China. Vaccination with this strain showed excellent immunogenicity and relatively moderate complications. The rates of post-vaccination side effects were significantly lower than other strains used in the global smallpox eradication campaign, including the NYCBH strain, which was utilized recently in the US against probable bioterrorism. Tiantan strain is characterized with low virulence and good immunogenicity. It also can induce more potent and long-lasting humoral and cellular responses than non-replicative vector.

Any suitable HIV antigen gene can be selected to construct a vaccine of this invention. For example, HIV antigen genes from the predominant prevalence strain can be selected based on the epidemiological study. In a specific embodiment of the invention, the B'/C subtype 97CN54 (CRF07) strain, which was first isolated and cloned in China was used as the target for vaccine construction. 97CN54 was found to be the most predominant HIV subtype causing more than 38.4% of infections in China. In the vaccine of the invention, at least one polynucleotide encoding HIV antigen(s) is inserted into TK region of the replicative vaccinia virus. Preferably, the inserted polynucleotide encodes HIV-1 antigens Gag, Pol and Env. Gag is a relatively conserved capsid protein of HIV. CTL and T helper responses against Gag have been identified in HIV-infected individuals (Van Baalen CA, et al. Fine- specificity of cytotoxic T lymphocytes which recognize conserved epitopes of the Gag protein of human immunodeficiency virus type-1. J Gen Virol 1996; 77: 1659-1665, Betts MR, et al. Cross-clade human immunodeficiency virus (HIV)-specific cytotoxic T-lymphcyte responses in HIV-infected Zambians. J Virol 1997; 71: 8908-8911, and Duradi D, et al. Cross-reactions between the cytotoxic T lymphocyte responses of human immunodeficiency virus-infected African and European patients. J Virol 1998; 72: 3547-3553). Env, the evelope protein of HIV, which is also the predominant target of neutralizing antibodies, is an essential component of the HIV vaccine. Although the Pol protein (the polymerase of HIV) is a less abundant protein, it contains many CTL and T helper epitopes and becomes more and more attractive as a potential immunogen (Walker BD et al. HIV-1 reverse transcriptase is a target for cytotoxic T lymphocytes in infected individuals. Science 1988 Apr 1; 240(4848):64-6). To enhance the safety and immunogenicity of the vaccine, some modifications and optimizations can be made to the polynucleotide encoding the HIV-1 antigen(s). For example, the sequence encoding the integrase and RNaseH activities can be deleted, so as to exclude the possibility of the foreign gene being integrated into the cellular genome. For the Env gene, Gp140TM region can be selected, which includes gp120 and the transmembrane region, through which the membrane protein can be anchored on the cell and thus the immunogenicity is enhanced. The methods for such modifications and optimizations are known for those skilled in the art.

In a specific embodiment of the invention, the polynucleotide encoding HIV gagpol has a nucleotide sequence as shown in SEQ ID NO:1, and the polynucleotide encoding HIV gp140TM has a nucleotide sequence as shown in SEQ ID NO:2.

To construct the vaccine of the invention, the polynucleotide encoding HIV antigen can be inserted into the TK region of vaccinia virus genome by any proper standard methods such as homologous recombination, so as to form a recombinant replicative vaccinia virus carrying the gene of interest. Preferably, the recombinant replicative vaccinia virus is free of selection marker gene.

In this context, this invention provides a universal shuttle vector to insert the gene of interest into TK region of vaccinia virus genome by way of recombination. In a specific embodiment, the universal shuttle vector is pVTT 1.0 (CGMCC No. 1458) containing the neo and lacZ selection marker genes. pVTT 1.0 includes the following elements: (1) Three selection marker genes: ampr, neo and lacZ. The reporter gene lacZ, which is under the control of the promoter P7.5, is used in blue/white plaque screening of recombinant vaccinia virus. Neo is under the control of the promoter PE6 for the purification of the recombinant vaccinia virus containing both selection marker and gene of interest. Through several rounds of plaque purification under the presence of G418 pressure, the purified recombinant vaccinia virus can be acquired (the growth of wild type recombinant vaccinia virus is restricted because of G418). The neo gene also has a poly(A) tail of 200bp to facilitate its function; (2) the flanking tkL and tkR fragments are fragments of the TK gene which ensure the intermolecular homologous recombination between the vaccinia virus and the shuttle vector; (3) lacZ' fragment which is 200bp in length and is completely homologous to the 200bp tail of the lacZ gene. The lacZ' fragment will ensure the intramolecular homologous recombination in the recombinant vaccinia virus containing both selection marker and gene of interest, and as a consequence, the selection marker genes will be removed; (4) vaccinia early/late pE/L promoter; and (5) multiple cloning sites, which are located downstream of pE/L promoter.

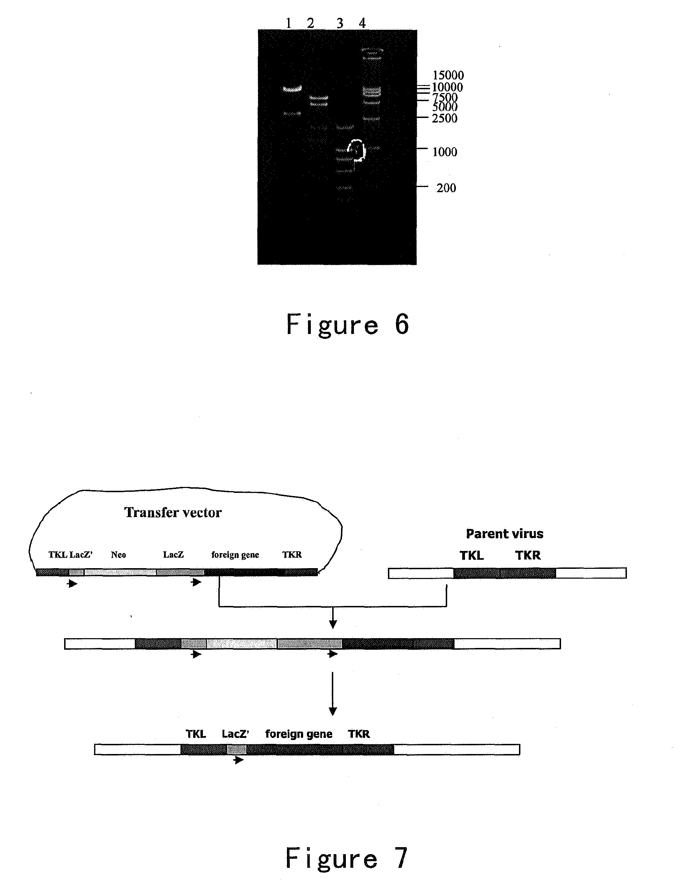

The universal shuttle vector pVTT1.0 allows the recombination of the gene of interest into TK region of vaccinia virus genome, and the obtained vaccinia virus genome is free of selection marker genes. Therefore, this invention also provides a method for developing an anti-SARS-CoV vaccine based on replicative vaccinia virus. In the method, the polynucleotide encoding HIV gagpol and gp140TM are placed into pVTT1.0 (CGMCC No. 1458) under the control of promoter pE/L to obtain a recombinant plasmid pVTT-gagpolenv. The pVTT-gagpolenv is then allowed to homologous recombination with vaccinia virus Tiantan on CEF cells, so that the HIV antigen gene and the selection marker genes LacZ and Neo can be inserted into the TK region of vaccinia virus genome DNA. In the presence of G418, the infected cells are covered by medium containing X-gal, neutral red and low melting point agarose. Blue plaques are picked up which contains the gene of interest and selection marker genes. This plaque purification is repeated for three rounds. And then under the condition without G418, intramolecular homologous recombination will take place in the recombinant vaccinia virus due to the existence of the small fragment of lacZ' in the shuttle vector and the intact lacZ gene. During this recombination, both lacZ and neo genes are removed but the sequence encoding HIV gagpol and gp140TM are retained in the genome of the recombinant vaccinia virus Tiantan strain. (Fig. 7).

The vaccine of this invention may further comprise a pharmaceutically acceptable adjuvant and/or carrier acceptable adjuvant, vector and/or excipient. Suitable adjuvant, vector and excipient are known for those skilled in the art.

In another aspect, the invention provides a vaccination regime comprising a primary immunization with HIV DNA vaccine (Ying Liu et al, Construction and Immune Potency of Recombinant Vaccinia Virus Expressing Polyvalent HIV Antigens. Chinese Journal of Virology, 2003, Vol 19(3):205-209) and a secondary immunization with the recombinant vaccinia virus Tiantan strain of the invention (prime-boost strategy). Such a strategy can elicit potent humoral and cellular responses and high-titer neutralizing antibodies.

The present invention will be further illustrated with reference to the following examples.

### Examples

### Example 1. Construction of vaccinia virus universal shuttle vector pVTT 1.0

### 1. The construction of recombinant plasmid pSC-neo

Plasmid pIRESneo (purchased from Clontech Company) was digested first with XhoI and then with SmaI (25°C), filled in with Klenow enzyme to form blunt ends, and then a fragment of 1.2kb named neo-polyA was obtained. Plasmid pSC65 (CGMCC No.1097) was digested with BglII, filled in with Klenow enzyme and treated with CIAP (Calf intestinal alkaline phosphatase), and then recovered by Agarose Gel DNA Fragment Recovery Kit. The two recovered fragments were ligated for 4h at 16°C and then used to transform E.coli Top 10. Separate clones were picked up and plasmid was extracted and then identified by XbaI and PstI digestion. The correct clone was named as pSC-neo.

### 2. Synthesis of the fusion fragment of early promoter PE6 and lacZ gene

Overlaping PCR was employed to synthesize the fusion fragment of promoter PE6 and LacZ gene. First, the sequences of both strands of the fusion fragment were listed and divided into 50bp fragments except that the most 5' fragment has a length of about 25bp, so that each 50bp fragment on the plus strand overlaps 25bp with the corresponding fragment on the negative strand. Secondly, deoxyoligonucleotides were synthesized according to the 50bp and 25bp sequence fragments described above. Thirdly, DNA fragments with lengths of about 500bp were generated by mixing 10 consecutive deoxyoligonucleotides with their complementary ones, heating and annealing in PCR tubes and then amplifying with both the most 5' deoxyoligonucleotides as PCR primers. Finally, the full-length fragment was generated by mixing the 500bp fragments, heating and annealing in a PCR tube and then amplifying with upstream and downstream primers. The fusion fragment of early promoter PE6 and lacZ gene was cloned into T-easy vector to create plasmid pT-lacZ'-PE6. The sequencing results proved the sequence to be correct (SEQ ID NO.7).

### 3. Construction of shuttle vector pVTT 1.0

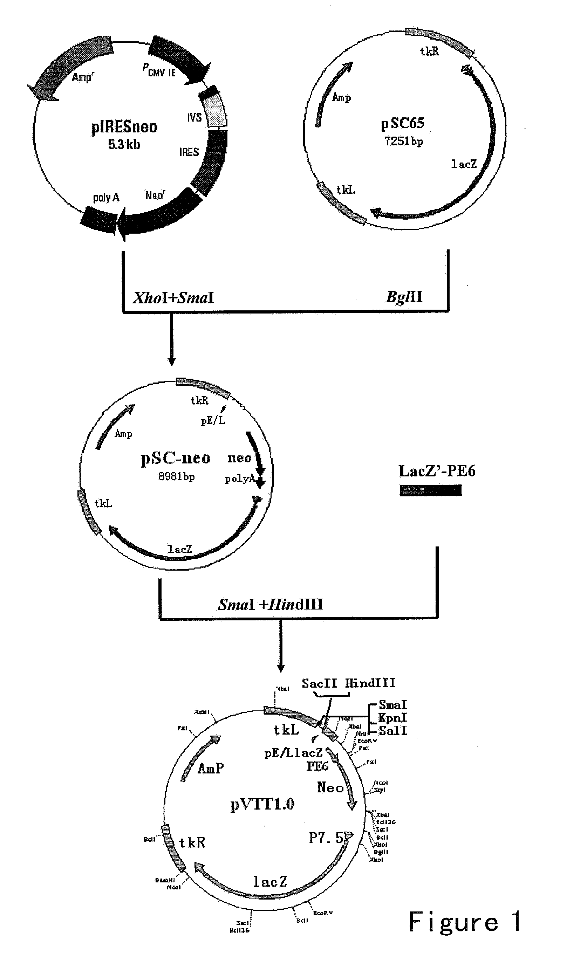

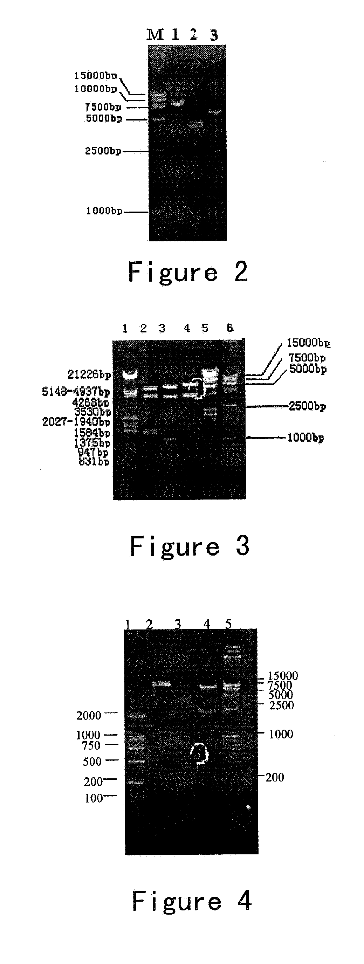

Plasmid pT-lacZ'-PE6 was digested with SmaI and HindIII and the 0.4kb fragment of lacZ'-PE6 was recovered. The shuttle vector pVTT 1.0 was obtained by inserting lacZ'-PE6 into the pSC-neo (Fig. 1). pVTT 1.0 was identified by KpnI, NdeI and EcoRV digestion. (Fig. 2)

### Example 2. Construction of transfer plasmid pVTT-gagpolenv

The construction of plasmid pVTT-gagpolenv includes the following three steps:

### 1. Construction of plasmid pVTT-gagpol

Plasmid pT-gagpol (CGMCC No.1438) comprising the gagpolΔ gene was developed by National Center for AIDS/STD Control and Prevention (NCAIDS), China CDC. pT-gagpol was digested by EcoRI and XmnI, filled in with Klenow to produce blunt ends and then a fragment of 2.9kb (gagpolΔ) was recovered. pVTT 1.0 was digested with SmaI, incubated with CIAP and a linear vector was then recovered. The obtained fragment was inserted into the obtained vector and then used to transform E. coli DH5α. Separate colonies were selected from which plasmid was extracted for restriction enzyme analysis. The correct clone was named pVTT-gagpol. The results of PstI, XbaI and NcoI digestion analysis of pVTT-gagpol is set out in Fig. 3.

### 2. Construction of plasmid pVTT-gp140TM

Plasmid pVTT-gp140TM (CGMCC No. 1439) comprising gp140TM gene was developed by National Center for AIDS/STD Control and Prevention (NCAIDS), China CDC. pT-gagpol was digested by SacI and SacII, filled in with Klenow to produce blunt ends and a fragment of 2.2kb named gp140TM was recovered. Vector pVTT 1.0 was digested with SmaI, incubated with CIAP and a linear vector pVTT 1.0 was then recovered. The obtained fragment was inserted into the obtained vector and then used to transform E. coli DH5α. Separate colonies were selected from which plasmid was extracted for restriction enzyme analysis with NdeI and SaII (Fig. 3). The positive clone was named pVTT-gp140TM.

### 3. Construction of transfer plasmid pVTT-gagpolenv

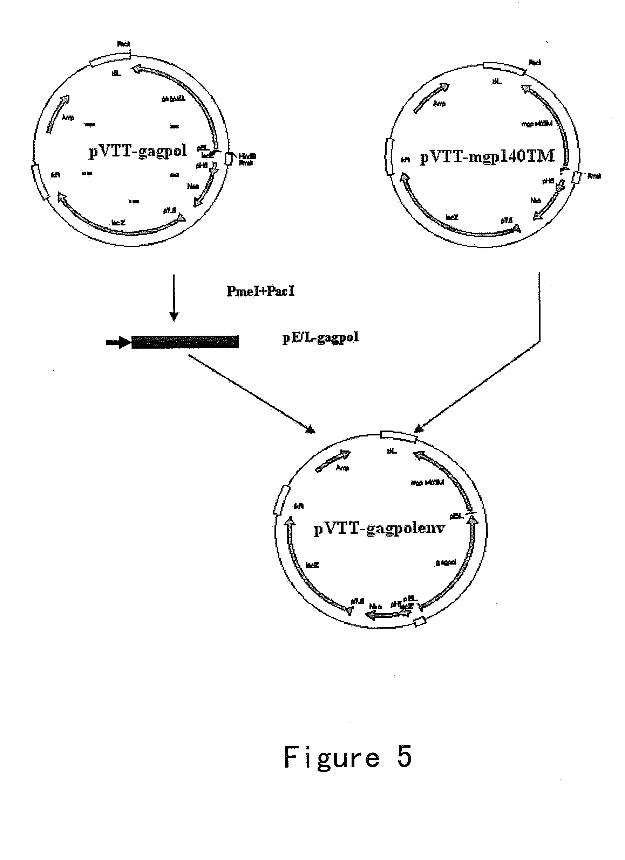

The construction scheme of plasmid pVTT-gagpolenv is shown in Fig. 5. Plasmid pVTT-gagpol was digested with PmeI and PacI, and filled in with Klenow enzyme to produce blunt ends. A fragment of 3.0kb thus obtained was named pE/L-gagpol. Plasmid pVTT-gp140TM was digested with PmeI and ligated with pE/L-gagpol, and then used to transform E. coli DH5α. The positive clone was selected and identified by HindIII and PstI restriction enzyme digestion (Fig. 6). The obtained plasmid was named pVTT-gagpolenv.

### Example 3. Screening of recombinant virus

The tkL and tkR regions of the transfer plasmid pVTT-gagpolenv allow homologous recombination occurring between the plasmid with the vaccinia virus Tiantan strain, and this causes the gagpolΔ and gp140TM genes to be inserted into the TK region of the vaccinia virus genome together with the marker genes neo and lacZ. In the presence of G418, the intramolecular homologous recombination will be inhibited because of the selection pressure, and thus marker genes LacZ and Neo will be temporarily retained in the virus genome. Blue plaques grow in the medium containing X-gal, neutral red and low melting point agarose can be picked up. The virus from the blue plaques contains both the genes of interest and the selection marker genes. The growth of non-recombinant virus will be inhibited in the presence of G418. In the subsequent selection without G418, the virus from the blue plaques will lose the lacZ and neo genes due to an intramolecular homologous recombination between a 200bp fragment of lacZ' in the transfer plasmid and the intact lacZ gene. During this secondary recombination, both lacZ and neo genes will be removed but the genes of interest will be retained in the genome of the recombinant vaccinia virus. The recombinant vaccinia virus containing only the genes of interest can be picked up as white plaques growing in low melting point agarose containing X-gal and neutral red. The screening strategy is shown in Fig. 7.

### 1. Transfection

CEF cells (80-90% confluent) were infected with vaccinia virus Tiantan strain (MOI=0.01∼0.1). After one hour at 37°C, the supernant was removed and the cells were washed twice with a serum free Eagle's medium. The virus infected CEF cells were then transfected with the transfer plasmid pVTT-gagpolenv according to the manufacturer's instructions (Invitrogen, Lipofectin Reagent Cat. 18292-91-011). After incubation for 48 hours at 37°C in CO₂ incubator, the virus solution was harvested by three cycles of freeze-thawing.

### 2. Screening of the virus

300µl of the viruses harvested in step (1) were used to infect confluent CEF cells which had been pre-treated with Eagle's medium containing 0.4mg/ml G418 for 24h. After incubated at 37°C in CO₂ incubator for 48h in the presence of 0.4mg/ml G418, the cells were covered by Eagle's medium containing 10mg/ml X-gal, 1% neutral red and 1% low melting point agarose. Two blue plaques occurred, which were respectively picked up and transferred into 1ml Eagle's medium. After three cycles of freeze-thawing, 100µl of the above virus was used to inoculate G418 pre-treated CEF cells. The above procedure was repeated. After 2 cycles of purification, 12 blue plaques of the third passage were obtained. The viruses thus obtained contain HIV genes of interest and selection marker genes lacZ and neo.

After three cycles of freeze-thawing, 100µl of each of the 12 blue plaques was inoculated to CEF cells which were not treated with G418, and incubated at 37°C in CO₂ incubator for 48h. The infected CEF cells were covered with low melting point agarose medium, progeny of ten blue viruses were blue and white plaques. 27 white plaques were picked up. After three cycles of freeze-thawing, the 27 white plaques were used to inoculate CEF on 24-well plate. 48h later, HIV antigen expression was detected by immune staining assay as follows.
a) removing the supernant after culturing for 48 hours;
b) fixing the cells with methanol:acetone solution (1:1) for 2 min;
c) washing twice with PBS;
d) diluting P24 antiserum (rabbit) with PBS containing 3% FBS at 1:1000;
e) adding to cells, 0.5ml of the diluted antiserum per well;
f) incubating at RT on shaker for 1h;
g) washing twice with PBS, 2 min for each;
h) adding a goat anti-rabbit antibody labelled by HRP (diluted with PBS containing 3% FBS at 1:5000);
i) incubating at RT on shaker for 30-45min;
j) washing twice with PBS, 2 min for each;
k) adding substrate DAB and incubating 5-10min at RT;
l) terminating the reaction.

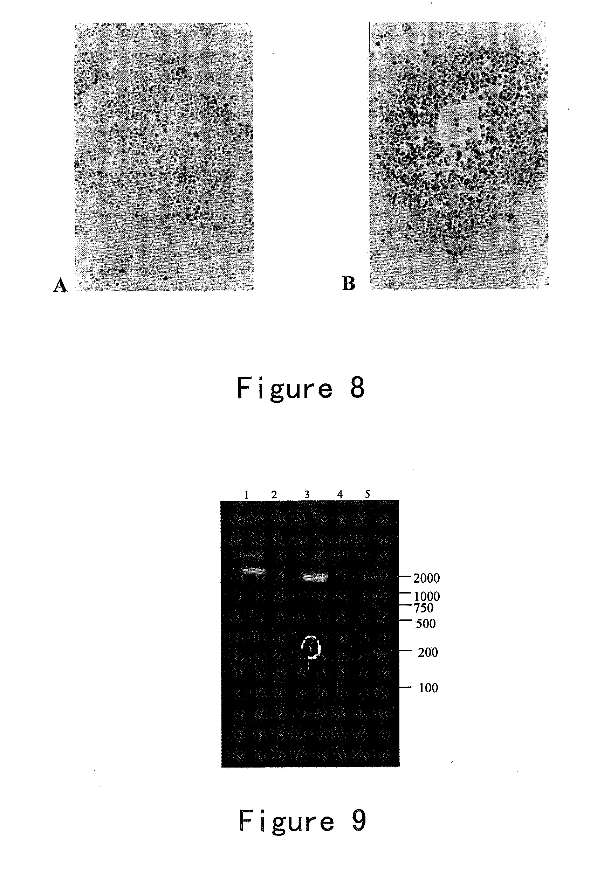

The results showed that viruses from 4 white plaques expressed HIV antigens (Fig. 8). These 4 plaques were used to inoculate CEF cells, and after three more rounds of purification and screening with antibody, the recombinant virus VTKgagpolenv was obtained.

### Example 4: Identification of recombinant virus VTKgagpolenv

The genomic DNA of the recombinant virus VTKgagpolenv and the Tiantan strain was extracted and used as the template for PCR amplification of gagpolΔ and mgp140TM genes. The primer sequences are as follows:
gagpol-for: 5'-GCG GAG GCT AGA AGG AGA GAG ATG G-3' (SEQ ID NO:3);
gagpol-rev: 5'-CTA CTA GCC TTC CAT GGC TAT TTT CTG CAC-3' (SEQ ID NO:4);
gp140-for: 5'-AGC GGA TCC ACC ATG AGA GTG ACG GGG ATC-3' (SEQ ID NO:5);
gp140-rev: 5'-GTC GGA TCC GAC TAA GGT CAG TAT CCT G-3' (SEQ ID NO:6).

The results showed that a 2.9kb fragment and a 2.1kb fragment were specifically amplified from VTKgagpolenv, demonstrating that the recombinant virus VTKgagpolenv contains the gagpolΔ and mgp140TM genes (Fig. 9).

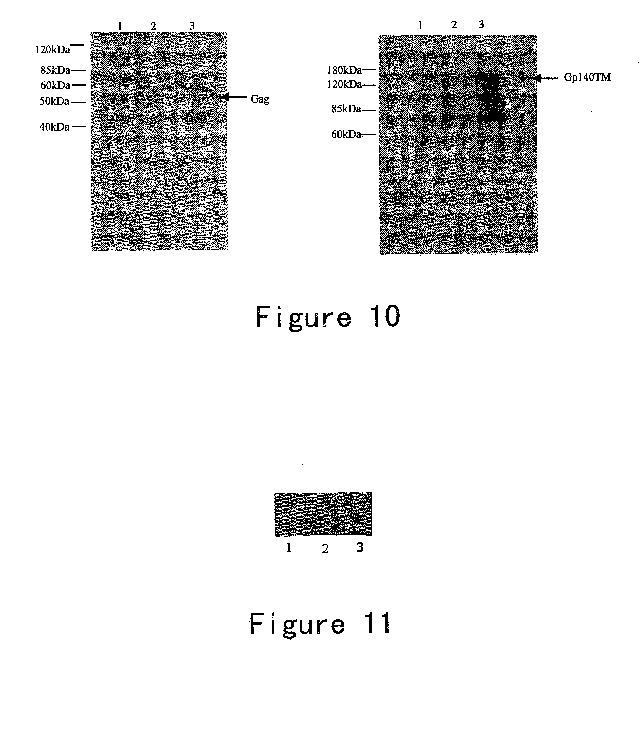

The expression of HIV antigens by VTKgagpolenv was detected by Western blotting. CEF cells were infected with VTKgagpolenv at dosages of 0.1∼0.01pfu/cell. After culturing for 48h, cells were harvested and then mixed with 1 ml of lysing buffer (20 mM Tris-Cl [pH 7.5], 1% SDS, 1%β-Mercaptoethanol, and 1 mM phenylmethylsulfonyl fluoride [PMSF]). The extracted proteins were subjected to PAGE and then transferred to an Immuno-Blot membrane. The membrane was blocked with 5% skim milk at RT and then incubated with 1:1000 diluted HIV-1 SF2 GP 160 antiserum (goat) and 1:2000 diluted HIV- 1 Gag P24 antiserum (rabbit) (NIH AIDS Research & Reference Reagent Programm) for 2 hours at RT. The membrane was washed for three times, each for 10 min. Protein-bound antibody was probed with horseradish peroxidase (HRP)-labeled secondary antibodies diluted at 1:5000 in 5% skim milk for 1 hour at RT. The membrane was washed for three times, each for 10 min. The results after visualization with DAB were shown in Fig. 10. The molecular weight of the Gag protein expressed by VTKgagpolenv is 55 kDa. An additional band of 41 kDa was also detected, which is an incompletely processed product of the Gag protein. The gp140TM protein expressed by VTKgagpolenv was within the range from 80 KDa to 140KDa, which represents the different molecular weights of gp140TM with varying degrees of glycosylation from non-glycosylation to fully glycosylation.

The DNA of the recombinant vaccinia VTKgagpolenv and Tiantan strain was extracted. Dot blot assays for determining loss of neo and lacZ genes were carried out following the protocols provided by the manufacturer (DIG High Prime DNA Labeling and Detection Starter Kit Cat. 1745832, Roche). The results showed that only the positive control produced positive dots, while both VTKgagpolenv and 752-1 were negative, indicating both neo and lacZ genes were deleted from VTKgagpolenv (Fig. 11).

The above results indicate that VTKgagpolenv could successfully express HIV antigens of interest, and the selection marker genes were completely removed. This demonstrates that VTKgagpolenv is the anti-AIDS vaccine based on recombinant vaccinia Tiantan strain.

### Example 5: The efficacy of the recombinant anti-AIDS vaccine to elicit HIV-specific immune responses in vivo

### 1. Rabbit model experiment

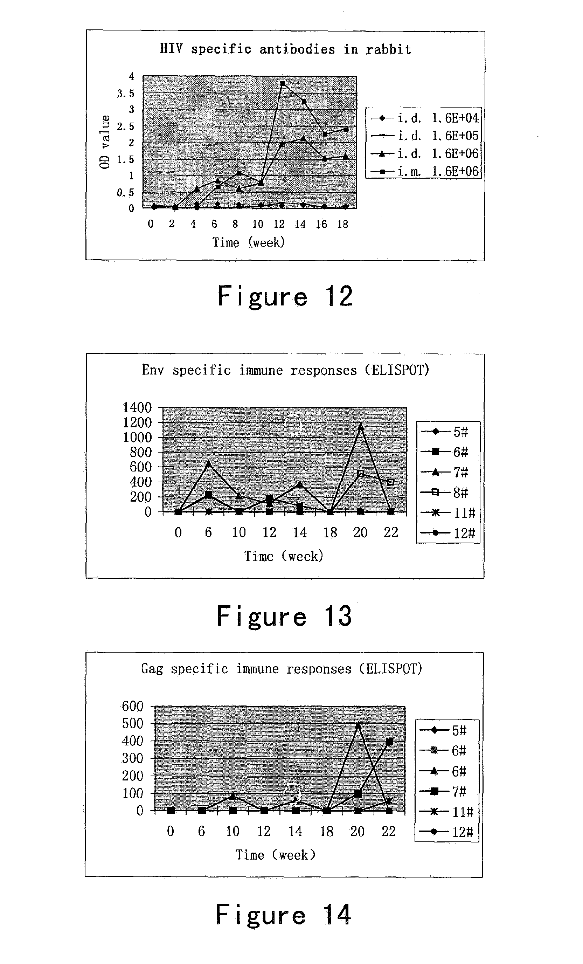

Three rabbits were vaccinated with VTKgagpolenv through percutaneous scarification in back with a dosage of 1.6×10⁴ pfu, 1.6×10⁵ pfu and 1.6×10⁶ pfu respectively at week 0 and week 10. One rabbit was injected with VTKgagpolenv (1.6×10⁶ pfu) intramuscularly at week 0 and week 10. Blood samples were collected from ear vein every two weeks after vaccination to detect the presence of HIV-specific antibody. Results shown in Fig. 12 indicated that HIV-specific antibody became detectable at week 4 in the rabbit vaccinated with a dosage of 1.6×10⁶ pfu, and the antibody level significantly increased after the boost immunization. The antibody levels induced in rabbits by vaccination with a dosage of 1.6×10⁴ pfu or 1.6×10⁵ were lower and lasted for a shorter duration. The rabbits vaccinated intramuscularly began to generate antibody after 6 weeks, and the level of antibody significantly increased after the boost immunization. The above experimental results demonstrate that HIV-specific antibodies can be induced in rabbits by VTKgagpolenv and a boost immunization can enhance immunogenic efficacy.

### 2. Mice model experiment

Female BALB/c(H-2d) mice (6-8 weeks old, 19-25g, purchased from National Institute for the Control of Pharmaceutical and Biological Products) were used to test the efficacy of the vaccine of the present invention. Mice in experimental group were injected intradermally with 50µl VTKgagpolenv (1.6 x 10⁷ pfu/ml) at multi-sites. Mice in control groups were injected with VTT at the same dosage. Intracellular IFN-γ secretion was tested after 6 weeks. Spleen of mice was harvested to prepare a cell suspension. The concentration of cells was adjusted to 5 x 10⁶, and seeded in a 96-well plate at 100 ul cells/well. Cells were stimulated with stimulating peptides (p24 peptide, env2 peptide pool and Pol peptide) at a final concentration of 5 µg/ml for 1h, followed by culturing for 18h with addition of BFA at a final concentration of 10 µg/ml. The cells was stained with CD8/PE, CD3/PE-cy5 and IFN-γ/FITC and then the ratio of IFN-γ secreting CD8 cells to the total CD8 cells was measured by flow cytometry. HIV-specific IFN-γ secreting cells induced by intradermal immunization of VTKgagpolenv comprised 0.659 % of the total CD8 cells, which was significantly different from the control group and demonstrated that VTKgagpolenv is capable of inducing HIV-specific cellular immunity in mice.

Five consecutive 2-fold dilutions of VTKgagpolenv (1.6 x 10⁷pfu/ml) were used. VTKgagpolenv were injected into Mice (20 mice/group) at bilateral tibialis anterior muscles. HIV-specific antibody level was tested at week 6. ELISA plate was coated with Gag protein (home made) with a coating concentration of 0.1 µg/ml. The primary antibody was the serum of the immunized mice diluted at 1:50; and the secondary antibody was HRP-labeled goat anti-mouse. Bliss method was used to calculate the ED₅₀. The ED₅₀ of the vaccine was 8.5×10⁵ pfu, indicating that VTKgagpolenv is capable of inducing HIV-specific cellular immunity in mice.

### 3. Monkey model experiment

Chinese-origin rhesus monkeys (Macaca mulatta) (about 3 years old, about 3 kg) were obtained from Primate Center, the Institute of Medical Biology, CAMS. There were four monkeys in the experimental group (5#-8#), and two monkeys in the control group (11#, 12#). The experimental group was vaccinated with a prime-boost strategy, in which 1 ml of mixed DNA vaccine containing 1mg of pGPNEF and 1mg of pGP140 (Ying Liu et al, Construction and Immune Potency of Recombinant Vaccinia Virus Expressing Polyvalent HIV Antigens. Chinese Journal of Virology, 2003, Vol 19(3):205-209) was injected intramuscularly at week 0, 4 and 10, and then boosted with intradermal injection of 5×10⁵ pfu of VTKgagpolenv at week 18. Control animals received vector DNA as well as VTT in the same way.

ELISPOT method was used to test HIV-specific cellular immune response. Peptides used in the test were obtained from NIH AIDS Research & Reference Reagent Program. The 87 peptides from Env region (Cat# 4830 to 4913) were divided into two peptide pools (env-1: 4830-4871 and env-2: 4872-4913); 121 peptides from Gag region (Cat# EVA 7080.1-7080.121) were divided into three peptide pools (gag-1: 7080.1-7080.40; gag-2; 7080.41-7080.80; and gag-3: 7080.81-7080.121). A commercial HIV antibody testing kit (biomerieux LTD.) was used to test HIV-specific antibody response.

The experimental results indicated that HIV specific humoral and cellular immune responses were induced in rhesus monkeys by the recombinant vaccinia virus. Particularly, a boost immunization with the recombinant vaccinia virus after the prime immunization with DNA vaccine significantly enhanced the strength of immune responses. Results were shown in Figs. 13, 14 and 15.

### Example 6: Protective effect of AIDS vaccine based on recombinant vaccinia Tiantan strain in SHIV challenging experiment

To determine whether the AIDS vaccine based on recombinant vaccinia Tiantan strain had an immuno-protective effect, the challenging test in monkey was performed using this vaccine. Rhesus monkeys (Macaca mulatta) (about 3 years old, about 3 kg) were obtained from Primate Center, the Institute of Medical Biology, CAMS. Four monkeys (1#-4#) in the experimental group (rTV) were vaccinated with 5 × 10⁵pfu of the recombinant vaccinia virus VTKgagpolenv at week 4, 18 and 65, and two monkeys (10# and 11#) in the control group were vaccinated with VTT at the same time. Additional four monkeys (5#-8#) in the experimental group (DNA+rTV) were treated with intramuscular injection of a mixed DNA vaccine containing 1mg of pGPNEF and 1mg of pGP140 at week 0, 4 and 10 (Ying Liu et al, Construction and Immune Potency of Recombinant Vaccinia Virus Expressing Polyvalent HIV Antigens. Chinese Journal of Virology, 2003, Vol 19(3):205-209), and then were vaccinated intradermally with 5×10⁵ pfu of the recombinant vaccinia virus VTKgagpolenv at week 18 and 65. Two monkeys in the control group (11#, 12#) received empty vector DNA as well as VTT in the same way.

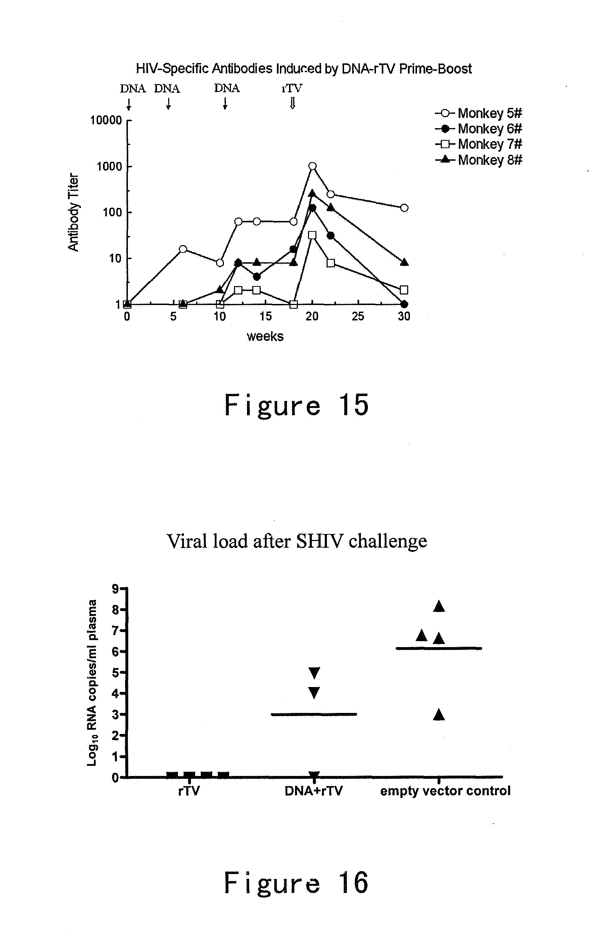

SHIV-CN97001 (CGMCC No. 1945) that was used as the challenging strain is a chimeric simian-human immunodeficiency virus (SHIV) that expresses the envelope protein from HIV B'/C recombinant strain CRF07 (HIV-CN97001). The animals were challenged at week 94 by intravenous injection of SHIV (128 TCID₅₀). In the group vaccinated three times with the recombinant vaccinia virus, all the four animals (1#-4#) were remained virus negative within 42 days from the challenge. In the DNA+rTV group (5#-8#), one (7#) remained virus negative within 21 days from the challenge, and two (6# and 8#) were found virus positive one day 14 post challenge, but the peak of virus load was lower than the mean value of control group by 2 logs. Another one in this group, i.e., the 5#, died of acute enteritis (confirmed by autopsy) 1 week prior to challenge. Among the 4 animals (9#∼12#) of the control group vaccinated with empty vector, SHIV replication was detected in 3 on day 14 post challenge. The above results suggest vaccination with the vaccine constructed based on the recombinant vaccinia Tiantan strain could provide complete protection against SHIV in vivo. Detailed data were given in Fig. 16.

The above embodiments and examples are merely illustrative of the principles of the inventions, and they should not be construed as limiting the scope of the invention by any means. It will be appreciated by one skilled in the art that other equivalent modifications and changes may be made without departing from the spirit of the inventions and the scope of the appended claims.

## Claims

1. A live vector AIDS vaccine expressing an HIV antigen(s), which vaccine comprises a replicative vaccinia virus as vector, wherein at least one polynucleotide encoding the HIV antigen(s) is inserted into the thymidine kinase gene (TK) region of the vaccinia virus genome.

2. The vaccine of claim 1, wherein the replicative vaccinia virus is vaccinia virus Tiantan strain.

3. The vaccine of claim 1 or 2, wherein the vaccine is free of any selection marker gene.

4. The vaccine of anyone of claims 1 to 3, wherein the at least one polynucleotide encoding the HIV antigen(s) is from HIV strain 97CN54 which is the predominant prevalence HIV strain in China.

5. The vaccine of anyone of claims 1 to 4, wherein the HIV antigen(s) being selected from gag-pol and gp140TM.

6. The vaccine of claim 5, wherein the polynucleotide encoding gp140TM has been modified by synonymous mutation and partial deletion, and has the nucleotide acid sequence as shown in SEQ ID NO: 1.

7. The vaccine of claim 5, wherein the polynucleotide encoding gag-pol has been modified by partial deletion, and has the nucleotide acid sequence as shown in SEQ ID NO: 2.

8. The vaccine of claim 6, wherein the polynucleotide encoding gag-pol has been modified by partial deletion, and has the nucleotide acid sequence as shown in SEQ ID NO: 2.

9. The vaccine of anyone of claims 1 to 8, further comprising a pharmaceutically acceptable adjuvant and/or carrier.

10. A method for preventing or treating HIV infection in a subject comprising administering to the subject an effective amount of the vaccine of anyone of claims 1 to 9.

11. The method of claim 10, comprising administering to the subject an effective amount of the vaccine of anyone of claims 1 to 9 for a plurality of times at regular intervals.

12. The method of claim 11, wherein the interval is from 14 to 47 weeks.

13. The method of anyone of claims 10 to 12, wherein the vaccine is administered in combination with another anti-HIV vaccine.

14. The method of claim 13, wherein the vaccine is administered ahead of, following, or simultaneously with the administration of said another anti-HIV vaccine.

## Amended claims

### Amended claims under Art. 19.1 PCT

**1.** A live vector AIDS vaccine expressing an HIV antigen(s), which vaccine comprises a replicative vaccinia virus Tiantan strain as vector, wherein at least one polynucleotide encoding the HIV antigen(s) is inserted into the thymidine kinase gene (TK) region of the vaccinia virus genome, and said polynucleotide has been modified by synonymous mutation and/or partial deletion.

**2.** The vaccine of claim 1, wherein the HIV antigen(s) is selected from gag-pol and gp 140TM of HIV strain 97CN54 which is the predominant prevalence HIV strain in China.

**3.** The vaccine of claim 2, wherein the polynucleotide encoding gp 140TM has the nucleotide acid sequence as shown in SEQ ID NO: 1.

**4.** The vaccine of claim 2 or 3, wherein the polynucleotide encoding gag-pol has the nucleotide acid sequence as shown in SEQ ID NO: 2.

**5.** The vaccine of any one of claims 1 to 4 for preventing or treating HIV infection in a subject, the preventing or treating comprising administering to the subject an effective amount of the vaccine..

**6.** The vaccine of any one of claims 1 to 4 for a medical use according to claim 5, the medical use comprising administering to the subject an effective amount of the vaccine of anyone of claims 1 to 4 for a plurality of times at regular intervals.

**7.** The vaccine of any one of claims 1 to 4 for a medical use according to claim 6, wherein the interval is from 14 to 47 weeks.

**8.** The vaccine of any one of claims 1 to 4 for a medical use according to any one of claims 5 to 7, wherein the vaccine is to be administered in combination with another anti-HIV vaccine.

**9.** The vaccine of any one of claims 1 to 4 for a medical use according to claim 8, wherein the vaccine is to be administered ahead of, following, or simultaneously with the administration of said another anti-HIV vaccine.
